# FASCICULE DE BREVET EUROPEEN

(11) **EP 3 972 953 B1**
(45) Date de publication et mention de la délivrance du brevet: **14.06.2023**
(21) Numéro de dépôt: 20734793.1
(22) Date de dépôt: 23.04.2020
(51) Int. Cl.: C07C 67/54, C07C 67/08, C07C 69/54

(54) **PROCEDE PERFECTIONNE DE FABRICATION D'ACRYLATES D'ALKYLE DE PURETE ELEVEE**
VERBESSERTES VERFAHREN ZUR HERSTELLUNG VON HOCHREINEN ALKYLACRYLATEN
IMPROVED METHOD FOR MANUFACTURING HIGH-PURITY ALKYL ACRYLATES

(30) Priorité: 23.05.2019 FR 1905414
(43) Date de publication de la demande: 30.03.2022
(73) Titulaire: ARKEMA FRANCE, 92700 Colombes (FR)
(72) Inventeur: TRETJAK, Serge, 57501 SAINT AVOLD cedex (FR); HILPERT, Camille, 57501 SAINT AVOLD cedex (FR)
(74) Mandataire: Arkema Patent
(86) Numéro de dépôt international: PCT/FR2020/050691
(87) Numéro de publication internationale: WO 2020/234519

(56) Documents cités:
- EP-A2- 2 659 943
- WO-A1-2016/016528

## Description

### DOMAINE TECHNIQUE

La présente invention concerne la fabrication de (méth)acrylates d'alkyle par estérification directe de l'acide (méth)acrylique par l'alcool correspondant.

Elle a pour objet un procédé de purification d'acrylate d'alkyle en C₄-C₁₀, en particulier d'acrylate de 2-éthylhexyle, conduisant à une productivité élevée d'un produit répondant aux normes en matière de pureté et d'acidité, dans des conditions énergétiques optimisées.

### ARRIERE-PLAN TECHNIQUE ET PROBLEME TECHNIQUE

L'estérification de l'acide (méth)acrylique est une réaction équilibrée avec génération d'eau qu'il est nécessaire d'éliminer au cours de la réaction pour déplacer l'équilibre dans le sens de la production de l'ester (méth)acrylique.

Les problèmes qui se posent lors de la fabrication des (méth)acrylates d'alkyle en C₄-C₁₀ par estérification directe de l'acide (méth)acrylique, généralement en présence d'une résine cationique comme catalyseur, sont le plus souvent liés à la complexité des étapes de purification nécessaires après l'étape réactionnelle pour obtenir un produit de haute pureté, au détriment en général de la productivité du procédé.

En effet, le problème qui se pose avec la mise en oeuvre de l'acide acrylique est celui de la formation d'acide β-hydroxypropionique (dénommé dans la suite « AHP »), et d'acide β-acryloxypropionique (dénommé dans la suite « dimère d'AA » ou « di-AA »).

L'AHP se forme vraisemblablement à partir du dimère d'AA en présence d'eau et au contact du catalyseur d'estérification. Sa formation dépend des conditions opératoires de réaction, de la nature du catalyseur utilisé, et de la quantité d'eau présente dans le milieu réactionnel.

En ce qui concerne les dimères d'AA, leur formation est pénalisante sur la quantité de sous-produits lourds à séparer et à incinérer, et en conséquence pénalisante pour la productivité.

Les mêmes types d'impuretés peuvent également se former dans le cas de l'acide méthacrylique, en particulier l'acide β-hydroxyméthylpropionique et l'acide β-méthacryloxypropionique.

Ces sous-produits posent un problème de pertes de matières premières et un problème de séparation pour être facilement éliminés, d'autant plus qu'ils peuvent former des azéotropes avec l'ester recherché. Il est donc difficile d'atteindre un ester (méth)acrylique de haute pureté.

En outre, la plupart des domaines d'application, notamment celui des adhésifs sensibles à la pression (PSA), requièrent la préparation de polymères (méth)acryliques à partir de monomères répondant à des normes strictes de pureté (> 99,7%) et exempts d'acidité, en particulier comportant une teneur en impuretés liées à l'acide (AHP+ di-AA ou AA) inférieures à 90 ppm.

Pour résoudre ces problèmes, le document WO 2016/016528 décrit des conditions opératoires permettant d'optimiser le rendement de la réaction et d'éliminer efficacement l'eau produite par la réaction, minimisant ainsi les réactions secondaires responsables de la formation d'impuretés acides et de sous-produits lourds. Ces conditions opératoires sont basées sur un excès d'alcool pour la réaction d'estérification et la mise en circulation d'une boucle réactionnelle ne comprenant que le réacteur d'estérification et une colonne de distillation éliminant l'eau produite sous forme d'un azéotrope avec l'alcool estérifiant. Ce procédé conduit à un ester purifié contenant de faibles traces d'impuretés liées à l'acide. Cependant, les solutions pour réduire l'AHP en cas de dérives de la réaction (augmentation de la teneur AHP en sortie de la boucle réactionnelle suite par exemple au vieillissement de la résine, à un changement de conditions opératoires ou à une moindre efficacité de la colonne de distillation) ne sont pas envisagées. En particulier, ces dérives de la réaction peuvent entraîner au niveau du train de purification, un surcoût énergétique important, et/ou l'ajout d'un décanteur et d'un évaporateur devant traiter l'ensemble du flux d'alimentation de la colonne de purification finale.

Avec le développement des colonnes de distillation à partition (connues sous la dénomination DWC- Divided wall column), des procédés de purification simplifiés sont également proposés pour produire des esters (méth)acryliques de haute pureté.

La demande de brevet EP 2 659 943 décrit une configuration d'une colonne à partition et son fonctionnement pour produire un acrylate de 2-éthylhexyle de haute pureté. Bien que cette colonne soit complexe à fabriquer et à opérer, elle présente l'avantage de réduire le coût d'équipement et la consommation d'énergie du processus de purification, comparativement à une installation conventionnelle comprenant deux colonnes de distillation. La question de la stabilisation nécessaire au bon fonctionnement de la colonne à partition et les problèmes liés à la séparation de l'AHP formant un azéotrope avec l'acrylate de 2-éthylhexyle ne sont cependant pas résolus.

Dans le document WO 2018/114429, une colonne à partition comportant une partie inférieure commune reliée à un rebouilleur unique est utilisée pour purifier l'acrylate de 2-éthylhexyle ou l'acrylate de 2-propylheptyle. Cependant la problématique de la formation d'AHP n'est pas abordée.

Il subsiste donc un besoin d'améliorer l'élimination des impuretés acides, en particulier l'élimination de l'acide β-hydroxypropionique (AHP) dans les procédés de synthèse/purification des esters acryliques décrits dans l'art antérieur.

Il a maintenant été découvert que la mise en oeuvre d'une colonne de distillation équipée d'un soutirage latéral et placée en sortie de la section réactionnelle permet de purger en continu de façon latérale une solution concentrée en AHP et dimères d'acide acrylique, et ainsi réduit la quantité d'impuretés acides résiduelles dans le produit purifié. De plus, en associant le soutirage latéral à un lavage de la solution soutirée par de l'eau, il est possible de recycler l'ester acrylique présent dans la phase soutirée, de façon à minimiser les pertes en produit.

La présente invention a donc pour objet un procédé de récupération/purification d'acrylate d'alkyle, simple à mettre en oeuvre, conduisant à un produit répondant aux normes en matière de pureté avec une productivité optimisée, tout en limitant la taille des équipements à mettre en oeuvre et le coût énergétique.

### RESUME DE L'INVENTION

L'invention a pour objet un procédé de récupération/purification d'un ester acrylique en C₄-C₁₀, à partir d'un mélange réactionnel brut obtenu par estérification directe de l'acide acrylique par l'alcool correspondant, caractérisé en ce qu'un flux riche en impuretés acides telles que l'acide β-hydroxypropionique et l'acide β-acryloxypropionique est soutiré par une sortie latérale lors de la distillation du mélange réactionnel brut.

Par « flux riche en impuretés acides », on entend que l'essentiel de ces impuretés acides générées lors de la réaction d'estérification est présent dans le flux qui est soutiré de façon latérale de la colonne de distillation alimentée par le mélange réactionnel brut. Ce flux comprend en outre de l'ester acrylique, des traces de réactifs non réagis et des sous-produits lourds de point d'ébullition supérieur à celui de l'ester acrylique, ainsi que des traces d'eau.

La Demanderesse a trouvé de façon surprenante que le profil de la colonne de distillation utilisée pour effectuer l'élimination des composés légers présents dans le mélange réactionnel brut (étêtage) présente un maximum de concentration (« ventre de concentration ») pour les impuretés acides, ce qui rend possible l'élimination desdites impuretés par soutirage latéral, à l'aide d'une colonne de distillation équipée d'un soutirage latéral.

Le flux riche en impuretés acides soutiré peut être sous forme gazeuse ou sous forme liquide, de préférence sous forme liquide.

Le soutirage latéral est de préférence effectué à un niveau plus bas que le niveau d'alimentation de la colonne de distillation, ce qui permet de minimiser la présence des réactifs valorisables tels que l'acide acrylique et l'alcool estérifiant, dans le flux soutiré. Selon un mode de réalisation du procédé selon l'invention, le flux riche en impuretés acides, soutiré latéralement, est soumis à un traitement avec de l'eau, afin de séparer lesdites impuretés acides et recycler le flux traité dans la colonne de distillation.

Selon un mode de réalisation, le flux traité exempt de l'essentiel des impuretés acides est recyclé dans la colonne de distillation.

Le flux traité exempt de l'essentiel des impuretés acides peut être recyclé dans la colonne de distillation à un niveau plus bas ou à un niveau plus haut que le soutirage latéral, de préférence il est recyclé à un niveau plus haut que le soutirage latéral.

De préférence, le flux traité exempt de l'essentiel des impuretés acides est recyclé dans la colonne de distillation à un niveau plus bas que le niveau d'alimentation de la colonne de distillation.

Le procédé selon l'invention est mis en oeuvre à l'aide d'un système de purification comprenant au moins une colonne de distillation équipée d'un soutirage latéral permettant la séparation de l'essentiel des impuretés acides présentes dans le mélange réactionnel brut. De préférence, ladite colonne de distillation est une colonne d'étêtage séparant en tête les composés légers tels que les réactifs non réagis présents dans le milieu réactionnel. L'invention a également pour objet un procédé de récupération/purification d'un ester acrylique en C₄-C₁₀, à partir d'un mélange réactionnel brut obtenu par estérification directe de l'acide acrylique par l'alcool correspondant, comprenant au moins les étapes suivantes :
i) on soumet le mélange réactionnel à un étêtage dans une colonne de distillation équipée d'un soutirage latéral permettant d'obtenir :
   - en tête un flux composé essentiellement des réactifs non réagis ;
   - en pied un flux comprenant l'ester recherché et des sous-produits lourds;
   - par soutirage latéral, un flux riche en impuretés acides ;
ii) on soumet le flux de pied de la colonne d'étêtage à une colonne de rectification permettant de séparer :
   - en tête l'ester recherché purifié ;
   - en pied un flux contenant des sous-produits lourds, qui est concentré sur un évaporateur à film ou distillé dans une colonne d'équeutage afin de recycler à la colonne d'étêtage les composés légers présents, et d'éliminer un résidu final de sous-produits lourds.

Le procédé selon l'invention peut comprendre en outre une étape iii) de traitement du flux soutiré latéralement :
iii) on soumet le flux riche en impuretés acides à une étape de lavage avec un flux aqueux permettant d'obtenir après décantation,
- une phase aqueuse comprenant la totalité des impuretés acides qui peut être envoyée à une station de traitement biologique ou en partie utilisée comme flux aqueux de lavage, et
- une phase organique, comprenant l'ester recherché, des sous-produits lourds et des traces d'eau et de réactifs, qui est recyclée au moins en partie dans la colonne d'étêtage. Le procédé selon l'invention permet d'obtenir un ester acrylique en C₄-C₁₀ de pureté supérieure ou égale à 99,7%, voire supérieure à 99,8%, et comportant une teneur en impuretés acides (AHP, dimère AA, AA) inférieure à 90 ppm, voire inférieure à 60 ppm. Dans son mode de réalisation préféré, comprenant en outre le recyclage après traitement du flux soutiré latéralement, l'invention conduit à une productivité optimisée tout en maintenant un bilan énergétique modéré.

L'invention s'applique avantageusement à la production d'acrylate de 2-éthylhexyle ou d'acrylate de 2-octyle, répondant aux normes de pureté exigées pour la production de polymères utilisables par exemple dans le domaine des adhésifs ou de revêtements.

Un autre objet de l'invention est un procédé de production d'un ester acrylique en C₄-C₁₀ exempt d'impuretés acides par estérification directe de l'acide acrylique par l'alcool correspondant comprenant le procédé de récupération/purification tel que défini ci-dessus.

D'autres caractéristiques et avantages de l'invention ressortiront mieux à la lecture de la description détaillée qui suit, en référence aux Figures 1, 2 et 3 annexées qui représentent :
[Fig. 1] : schéma de principe d'un premier procédé de purification selon l'art antérieur.
[Fig. 2] : schéma de principe d'un second procédé de purification selon l'art antérieur.
[Fig. 3] : schéma de principe du procédé selon l'invention.

### DESCRIPTION DETAILLEE DE L'INVENTION

L'invention est basée sur la mise en oeuvre d'une purge d'un flux riche en impuretés acides à l'aide d'un soutirage latéral équipant de préférence une colonne d'étêtage dans un procédé de purification d'un mélange réactionnel brut obtenu par estérification directe de l'acide acrylique par un alcool en C₄-C₁₀.

L'alcool estérifiant peut être un alcool aliphatique primaire ou secondaire, comportant une chaine alkyle linéaire ou ramifiée comportant de 4 à 10 atomes de carbone. Comme exemples d'alcools, on peut citer le butanol, le 2-éthyl hexanol, le n-octanol, le 2-octanol, le n-décanol et 2-propyl heptanol.

De préférence, l'alcool est le 2-éthyl hexanol ou le 2-octanol.

La réaction d'estérification est effectuée généralement dans un réacteur surmonté d'une colonne de distillation permettant d'extraire l'eau générée par la réaction. L'eau de réaction est éliminée au fur et à mesure de sa formation sous forme d'azéotrope avec l'alcool estérifiant afin de déplacer l'équilibre d'estérification.

Les conditions opératoires de la réaction d'estérification ne sont pas critiques, le procédé selon l'invention pouvant s'appliquer au mélange réactionnel quel que soit son procédé d'obtention. Ainsi la réaction peut être effectuée en excès d'acide ou en excès d'alcool, à une température généralement comprise entre 70° et 100°C, de préférence entre 75°C et 95°C.

Le réacteur peut être un réacteur à lit fixe ou un réacteur à lit en suspension. La colonne de distillation, surmontant le réacteur est en général à garnissage et elle est équipée d'un condenseur de tête, un décanteur, permettant de décanter les vapeurs condensées en tête et de séparer une phase organique comprenant de l'alcool et des traces d'ester, qui est recyclée dans la colonne, et une phase aqueuse qui est éliminée. La colonne fonctionne généralement à une pression allant de 6,6 à 9,3 kPa (50 à 70 mmHg).

Comme catalyseur d'estérification, on utilise généralement une résine cationique, de préférence une résine cationique forte, par exemple une résine cationique forte sulfonée de type styrène/divinyl benzène à groupements sulfoniques. A titre d'exemple de résines, on peut citer celles commercialisées sous les dénominations DIAION^{®} PK208 ou PK216 par la société Mitsubishi, ou celles commercialisées sous la dénomination LEWATIT^{®} K2620 ou K2621 par la société Lanxess, ou celles commercialisées sous la dénomination AMBERLYST^{®} A15, A16 ou A46 par la Société Rohm & Haas.

On conduit la réaction d'estérification généralement en présence d'au moins un inhibiteur de polymérisation choisi parmi la phénothiazine, l'hydroquinone (HQ), et ses dérivés tels que l'éther méthylique de l'hydroquinone (EMHQ), le 2,6-di-terbutyl-4-méthyl phénol (BHT), le 2,4-diméthyl-6-terbutyl phénol (Topanol A), les sels de l'acide thiocarbamique ou dithiocarbamique, les composés N-oxyls, comme le 4-hydroxy-2,2,6,6-tétraméthyl pipéridinoxyl (4-OH Tempo), les composés à groupements nitroso, tels que la N-nitroso phényl hydroxylamine et ses sels d'ammonium, les quinones telles que la benzoquinone, et les composés aminés comme les dérivés de paraphénylènediamine, à des teneurs dans le milieu de réaction pouvant être comprises entre 50 ppm et 5000 ppm, éventuellement en présence d'air appauvri, mais généralement à des teneurs comprises entre 150 ppm et 1000 ppm. L'ajout des inhibiteurs de polymérisation peut se faire à différents endroits, avec l'introduction des réactifs ou en tête de colonne de distillation.

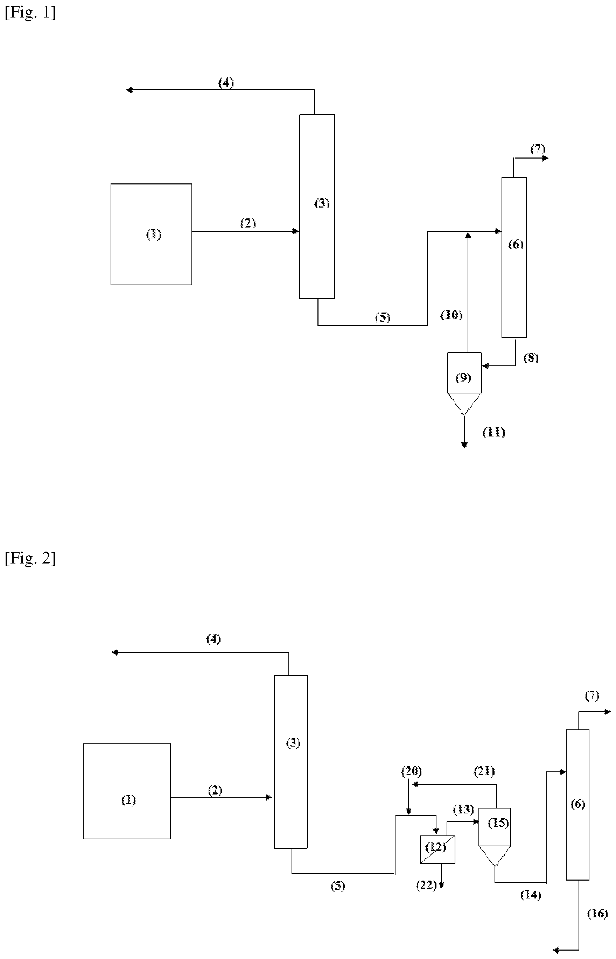

En référence à la Figure 1 qui représente le schéma de principe d'un procédé de récupération/purification d'un ester acrylique selon l'art antérieur, le mélange brut réactionnel (2) sortant de la zone réactionnelle (1) est envoyé sur une colonne d'étêtage (3) qui sépare, en tête, un flux (4) comprenant essentiellement les réactifs non réagis, et en pied, un flux (5) comportant principalement l'ester recherché avec des impuretés liées à l'acide et à l'alcool et des sous-produits lourds. La colonne (3) est par exemple une colonne à plateaux, type plateaux perforés, ou à garnissage. Le flux (5) est envoyé sur une colonne de rectification (6) conduisant en tête à un flux (7) d'ester purifié, et en pied à un flux (8) qui est concentré sur un évaporateur à film (9) ou distillé dans une colonne d'étêtage (non représentée) afin de recycler les composés légers (10) présents vers le début de la section de purification, tels que des traces de réactifs non réagis, et d'éliminer le résidu (11) final de produits lourds.

Le flux (4) comprend essentiellement les réactifs non réagis, acide acrylique et alcool estérifiant, qui sont séparés de l'ester recherché en raison de leur point d'ébullition plus faible. Ce flux (4), valorisable, est recyclé à la réaction.

Selon ce procédé de l'art antérieur représenté à la Figure 1, il est possible d'adapter les conditions de fonctionnement de la colonne d'étêtage (3) de façon à entraîner l'AHP formé dans le flux de tête recyclé à la réaction. Toutefois, même si ce mode de réalisation permet de limiter la quantité d'impuretés acide dans l'ester purifié, il entraine, soit un accroissement énergétique au bouilleur de la colonne d'étêtage si on souhaite maintenir constant le débit de pied vers la colonne (6) de purification de l'ester, soit une baisse de production en diminuant le débit de pied de cette colonne d'étêtage (3) vers la colonne de purification (6) pour un coût énergétique constant.

En référence à la Figure 2 qui représente le schéma de principe d'un second procédé de récupération/purification d'un ester acrylique selon l'art antérieur, un lavage à l'eau du mélange réactionnel (2) après étêtage dans la colonne (3) est effectué avant de séparer les impuretés et les sous-produits lourds dans la colonne de rectification (6). Selon ce mode de réalisation, le flux de pied (5) de la colonne d'étêtage est soumis à un lavage avec un flux aqueux (20) conduisant, après décantation dans un décanteur (12), à une phase aqueuse (22) comprenant la totalité des impuretés acides ainsi qu'à une phase organique (13) comprenant l'ester recherché, des sous-produits lourds et des traces d'eau et d'acide acrylique.

La phase organique (13) obtenue après lavage à l'eau est soumise à une étape d'élimination d'eau, par distillation à l'aide d'une colonne à distiller ou à l'aide d'un évaporateur à film mince (15), l'eau récupérée (21) pouvant être recyclée à l'étape de lavage.

Le flux exempt d'eau (14) est ensuite envoyé vers la dernière colonne de rectification (6) de l'ester pur, avec élimination en pied d'un flux (16) de sous-produits lourds.

Dans ces conditions, le produit distillé (7) en tête de colonne (6) est un ester purifié ne contenant sensiblement plus d'impuretés acides. Cependant, outre le fait qu'il faille un décanteur de grand volume pour traiter l'ensemble du flux (5), il est nécessaire de traiter par passage sur un évaporateur (15) une partie du flux lavé. Ce mode de réalisation accroît très fortement la consommation énergétique et est difficilement compatible avec un procédé à l'échelle industrielle.

L'invention remédie aux inconvénients desdits procédés de l'art antérieur, par l'utilisation d'une colonne de distillation équipée d'un soutirage latéral comme colonne d'étêtage (3).

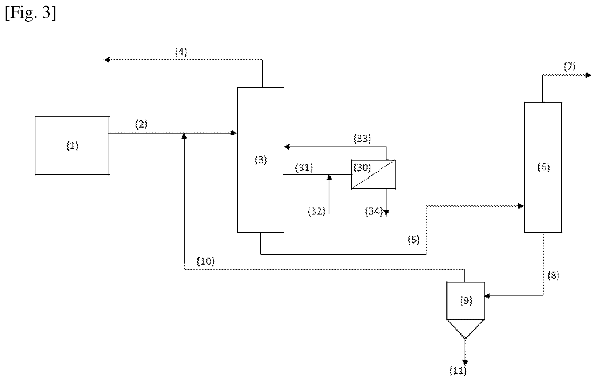

Selon le schéma de principe du procédé selon l'invention, représenté à la Figure 3, le mélange réactionnel (2) alimente une colonne d'étêtage à soutirage latéral (3).

Les internes utilisés pour la colonne (3) peuvent être des plateaux à clapets ou des plateaux perforés à déversoir, ou des plateaux à courants croisés comme les Dual Flow, les Ripple Trays, les Turbo Grid Shell, ou du garnissage ordonné comme du garnissage structuré tel le Mellapack 250X de Sulzer.

La colonne d'étêtage (3) comporte avantageusement un équivalent de 10 à 30 plateaux théoriques de préférence de 15 à 20 étages théoriques.

L'alimentation de la colonne d'étêtage (3) par le flux (2) est réalisée généralement au tiers supérieur de cette colonne, de préférence entre les plateaux théoriques 3 à 10 comptés à partir de la tête de la colonne.

La colonne fonctionne avec un taux de reflux (débit de liquide condensé retourné à la colonne/ débit (4) extrait en tête) avantageusement compris entre 115 et 1/1, de préférence de l'ordre de 1/3.

La colonne (3) peut opérer sous vide, afin de minimiser l'exposition thermique des composés thermosensibles au sein de la colonne. Avantageusement, la colonne (3) fonctionne sous un vide allant de 1,333 à 13,332 kPa (10 à 100 mm de Hg).

Le flux (4) de tête de la colonne (3) comprend essentiellement les réactifs non réagis. Ce flux (4) valorisable, est avantageusement recyclé à la réaction.

Selon l'invention, un flux (31) est soutiré latéralement de la colonne (3). Ce flux (31) peut être sous forme gazeuse ou sous forme liquide, de préférence sous forme liquide. Le soutirage est placé à un niveau plus bas que le niveau d'alimentation de la colonne, avantageusement entre les plateaux théoriques 5 à 15, de préférence entre 8 et 12 comptés à partir de la tête de colonne. L'emplacement de ce soutirage latéral est judicieusement choisi de façon à maximiser la concentration d'AHP et celle de di-AA tout en minimisant la présence de réactifs valorisables (Acide acrylique et alcool estérifiant). Ce soutirage latéral comprend généralement la quantité de stabilisants nécessaires à son fonctionnement sans encrassement. On peut si besoin en cas de soutirage en phase gazeuse ajouter également un autre stabilisant. Avantageusement, on introduit de 100 à 5000 ppm d'inhibiteur de polymérisation dans le système de purification selon le procédé de l'invention. Les inhibiteurs de polymérisation employés peuvent être identiques à ceux utilisés pour stabiliser la réaction d'estérification.

Selon un mode de réalisation, la stabilisation de la colonne d'étêtage (3) est réalisée à l'aide d'un premier inhibiteur de polymérisation, de préférence injecté au niveau du condenseur de tête. L'ester acrylique ainsi que l'AHP soutiré latéralement de la colonne sous forme d'un flux gazeux ou en flux liquide peut être stabilisé avec un inhibiteur de polymérisation différent du premier inhibiteur. Le flux organique (33) pourra être également stabilisé avec le premier inhibiteur avant d'être ré-introduit dans la colonne d'étêtage.

Pour rendre les inhibiteurs plus efficaces, on peut injecter en pied de la colonne (3) de l'oxygène, de l'air ou de l'air dit appauvri à 7% O₂. De façon préférée la quantité d'oxygène injectée correspond à une teneur de 0,2% à 0,5% rapportée à la quantité de vapeur organique dans la colonne.

Le flux soutiré (31) est envoyé dans un décanteur (30) dans lequel on additionne de l'eau (32). Après refroidissement à une température de préférence allant de 20°C à 70°C, de l'eau (32) est additionnée dans une proportion généralement comprise entre 5 et 50% par rapport au flux (31) provenant du soutirage latéral.

Après lavage et décantation des phases, la phase organique (33) est réintroduite dans la colonne (3), à un niveau qui peut être inférieur ou supérieur à celui du soutirage latéral, de préférence à un niveau supérieur à celui du soutirage latéral, et de préférence à un niveau plus bas que l'alimentation de la colonne, en particulier entre les plateaux théoriques 5 à 15, de préférence 7 à 12. La phase aqueuse (34) comprenant l'essentiel des impuretés acides peut être à nouveau réutilisée pour laver le flux 31, ou envoyée pour traitement à une station biologique.

Le flux (5), séparé en pied de la colonne d'étêtage (3) est envoyé sur une colonne de rectification (6) conduisant en tête à un flux (7) d'ester purifié et en pied à un flux (8).

La colonne (6) est par exemple une colonne à plateaux perforés ou à garnissage. Les internes utilisés pour la colonne peuvent être des plateaux à clapets ou des plateaux perforés à déversoir, ou des plateaux à courants croisés comme les Dual Flow les Ripple Trays, les Turbo Grid Shell, ou du garnissage ordonné comme du garnissage structuré tel le Mellapack 250X de Sulzer.

La colonne de distillation (6) comporte avantageusement un équivalent de 2 à 15 plateaux théoriques, de préférence de 5 à 10 étages théoriques.

La colonne (6) fonctionne avec un taux de reflux (débit de liquide condensé retourné à la colonne/ débit du flux (7)) al1ant de 1/5 à 1/1, de préférence de l'ordre de 1/2.

La colonne (6) peut opérer sous vide, afin de minimiser l'exposition thermique des composés thermosensibles au sein de la colonne. Avantageusement, la colonne (6) fonctionne sous un vide allant de 1,333 à 13,332 kPa (10 à 100 mm de Hg). Avantageusement, sa température de fonctionnement est comprise entre 50°C et 160 °C. Le flux (8) séparé en pied est concentré sur un évaporateur à film raclé (9) afin de recycler les composés légers présents vers le début de la section de purification en amont de la colonne (3) ou de la colonne (6) et permet d'éliminer le résidu (11) comportant les produits lourds.

Selon l'invention, le flux (7) de tête de la colonne (6) est constitué de l'ester recherché ayant comme spécifications une pureté en ester supérieure à 99,7%, une teneur en impuretés acides (AHP+ diAA +AA) inférieure à 90 ppm. La teneur en eau est généralement inférieure à 400 ppm.

Les exemples ci-après illustrent la présente invention sans toutefois en limiter la portée.

### PARTIE EXPERIMENTALE

Dans les exemples, les pourcentages sont indiqués en poids sauf indication contraire et les abréviations suivantes ont été utilisées :
AA : acide acrylique
A2EH : acrylate de 2-éthylhexyle
2EH : 2-éthyl hexanol
AHP : acide β-hydroxypropionique
Di-AA : dimères d'AA
PTZ : phénothiazine

Dans la partie expérimentale, en référence aux Figures 1 et 2 selon l'art antérieur et à la Figure 3 selon l'invention, les colonnes 3 et 6 ont une configuration fixée.

Les caractéristiques principales de ces deux colonnes sont les suivantes :
Colonne 3 : 45 plateaux de type Dual Flow (comptés à partir de la tête de colonne)
   Pression tête de colonne : 3,3 kPa (25 mmHg).
   Alimentation : plateau 15
   Air : plateau 45
   Stabilisant PTZ : plateau 1
Colonne 6 : 25 plateaux de type Dual Flow (comptés à partir de la tête de colonne)
   Pression tête de colonne : 2,7 kPa (20 mmHg)
   Alimentation : plateau 25
   Air : plateau 25
   Stabilisant PTZ : plateau 1

### Exemple 1 (Référence)

Le flux 2 d'alimentation d'une colonne d'étêtage (3) d'un procédé de purification d'acrylate de 2-éthylhexyle représenté à la Figure 1, contient 75 ppm d'AHP dans un premier essai, et 140 ppm d'AHP dans un second essai.

Les conditions de mise en oeuvre du procédé et la composition des différents flux sont résumées dans le tableau 1 ci-après.

Dans les conditions de l'essai 1 où la teneur en AHP dans le flux d'alimentation du train de purification est de 75 ppm, l'installation représentée à la figure 1 permet de fabriquer 4950 kg/h, soit environ 119 t/j de A2EH avec une pureté de 99,7% et comportant des composés acides (di-AA+ AA+ AHP) à une teneur de 84 ppm. Ce produit répond aux spécifications commerciales.

L'énergie engagée dans cet essai au niveau du bouilleur des colonnes 3 et 6 et de l'évaporateur 9 est de 2222618 kcal/h, correspondant à 9299433 kJ/h.

Dans les conditions de l'essai 2 où la teneur en AHP dans le flux d'alimentation du train de purification est de 140 ppm, pour une énergie engagée au niveau du bouilleur des colonnes 3 et 6 et de l'évaporateur 9 sensiblement équivalente (2222813 kcal /h) l'installation représentée à la figure 1 ne permet pas de fabriquer un A2EH répondant aux spécifications relatives à la teneur en composés acides (< 90 ppm), la teneur globale en Di-AA + AA + AHP dans le flux 7 étant de 156 ppm, et la pureté du A2EH de 99,6% est hors spécification.

**[Tableau 1]**

| | Flux | 2 | 4 | 5 | 7 | 11 |
|---|---|---|---|---|---|---|
| | Température, °C | 115 | 29,7 | 143,1 | 23,8 | 138 |
| | Débit massique, kg/hr | 13600 | 7570,199 | 6300 | 4949,999 | 331,745 |
| Essai 1 | Fraction massique | | | | | |
| | AA | 0,065 | 0,119 | 0 | 0 | 0 |
| | 2EH | 0,231 | 0,423 | 0 | 0 | 0 |
| | A2EH | 0,649 | 0,405 | 0,941 | 0,997 | 0,326 |
| | EAU | 0,005 | 0,008 | 0 | 0 | 0 |
| | Di-AA | 0,001 | 10 ppm | 0,002 | 29 ppm | 0,007 |
| | AHP, ppm | 75 | 99 | 47 | 55 | 15 |
| Essai 2 | AA | 0,065 | 0,119 | 0 | 0 | 0 |
| | 2EH | 0,231 | 0,423 | 0 | 0 | 0 |
| | A2EH | 0,649 | 0,406 | 0,94 | 0,996 | 0,325 |
| | EAU | 0,005 | 0,008 | 0 | 0 | 0 |
| | Di-AA | 0,001 | 10 | 0,002 | 29 | 0,007 |
| | AHP, ppm | 142 | 170 | 108 | 127 | 35 |

### Exemple 2 (comparatif)

L'essai 2 de l'exemple 1 est reproduit en changeant certaines conditions opératoires afin de retrouver un produit répondant aux spécifications.

Ainsi, dans l'essai 3, le débit massique du flux 4 distillé en tête de la colonne d'étêtage 3 est augmenté, avec pour conséquence une baisse du débit de production en A2EH (flux 7 en tête de la colonne 6).

Dans l'essai 4, le débit de rebouillage de la colonne d'étêtage 3 est augmenté, avec pour conséquence une augmentation de la consommation énergétique du procédé de purification.

Les résultats sont rassemblés dans le tableau 2.

Les conditions de l'essai 3 conduisent à un produit répondant aux spécifications, avec une consommation énergétique légèrement plus faible (2209593 kcal/h, correspondant à 9244937 kJ/h) que l'essai 1.

Cependant, une perte en productivité est constatée (107 t/j au lieu de 119 t/j).

Les conditions de l'essai 4 génèrent un A2EH conforme aux spécifications et pour une productivité équivalente à celle de l'essai 1, mais avec une surconsommation énergétique de l'ordre de 10%.

**[Tableau 2]**

| | Flux | 2 | 4 | 5 | 7 | 11 | 8 |
|---|---|---|---|---|---|---|---|
| | Température, °C | 115 | 29,7 | 143,1 | 23,8 | 138 | 144,3 |
| Essai 3 | Débit massique, kf/hr | 13600 | 8070,384 | 5800 | 4449,998 | 331,723 | |
| | Fraction massique | | | | | | |
| | AA | 0,065 | 0,111 | 0 | 0 | 0 | |
| | 2EH | 0,231 | 0,396 | 0 | 0 | 0 | |
| | A2EH | 0,649 | 0,442 | 0,937 | 0,998 | 0,326 | |
| | EAU | 0,005 | 0,008 | 0 | 0 | 0 | |
| | Di-AA | 0,001 | 11 | 0,002 | 22 ppm | 0,007 | |
| | AHP, ppm | 140 | 211 | 41 | 50 | 13 | |
| Essai 4 | Débit massique, kg/hr | 13600 | 7570,203 | 6300 | 4949,999 | 331,963 | 1350 |
| | Fraction massique | | | | | | |
| | AA | 0,065 | 0,119 | 0 | 0 | 0 | 0 |
| | 2EH | 0,231 | 0,423 | 0 | 0 | 0 | 0 |
| | A2EH | 0,649 | 0,405 | 0,942 | 0,998 | 0,326 | 0,735 |
| | EAU | 0,005 | 0,008 | 0 | 0 | 0 | 0 |
| | Di-AA | 0,001 | 9 | 0,002 | 29 | 0,007 | 0,007 |
| | AHP, ppm | 140 | 220 | 44 | 52 | 14 | 15 |

### Exemple 3 (comparatif)

En référence à la figure 2, le flux 5 de pied de la colonne d'étêtage 3 est envoyé dans un décanteur 12 et est lavé avec 20% d'eau à 70°C. Le flux organique 13 est repris par un évaporateur à film 15 avant d'être soumis à la distillation dans la colonne 6 : le flux aqueux 22 est envoyé à un traitement biologique et le flux de tête 21 de l'évaporateur est renvoyé à l'alimentation du décanteur 12.

La composition des différents flux est résumée dans le tableau 3.

**[Tableau 3]**

| Flux | 2 | 4 | 5 | 20 | 22 | 14 | 21 | 16 | 7 |
|---|---|---|---|---|---|---|---|---|---|
| Température, °C | 115 | 29,7 | 143 | 20 | 20 | 110 | 110 | 138 | 23,8 |
| Débit massique, kg/hr | 13600 | 7570,284 | 6300 | 1260 | 1471,025 | 6317,276 | 432,724 | 17,03 | 4967,236 |
| Débit vol cumulé/hr | 16,88 | 8,627 | 7,991 | 1,262 | 1,499 | 7,777 | 2389,327 | 0,017 | 5,631 |
| Fraction massique, | | | | | | | | | |
| AA | 0,063 | 0,116 | 0 | 0 | 0 | 0 | 0 | 0 | 0 |
| 2EH | 0,225 | 0,412 | 0 | 0 | 0 | 0 | 0 | 0 | 0 |
| A2EH | 0,658 | 0,42 | 0,943 | 0 | 0 | 0,976 | 0,963 | 0,316 | 0,996 |
| EAU | 0,005 | 0,008 | 0 | 1 | 0,856 | 0 | 0,031 | 0 | 0 |
| Di-AA, ppm | 181 | 3 | 395 | 0 | 10 | 0 | 0 | 0 | 0 |
| AHP, ppm | 136 | 186 | 75 | 0 | 315 | 0 | 0 | 0 | 0 |

Le traitement global du flux 5 permet de réduire à 0 ppm la teneur en composés acides dans le flux alimentant la colonne 6. Il en résulte en tête de colonne un flux 7 distillé exempt de composés acides. Cependant, la pureté 99,6% du A2EH distillé ne satisfait pas la spécification en terme de pureté.

Par ailleurs, le coût énergétique de cette opération est de 18% supérieure à celui nécessaire pour un procédé sans lavage intermédiaire.

### Exemple 4 (selon l'invention)

On reproduit l'exemple 1 (essai 2), mais dans une configuration selon l'invention (représentée sur la Figure 3). Le flux d'alimentation (2) contient 140 ppm de AHP.

Le flux 31 est soutiré au plateau 26 de la colonne 3 et lavé dans un décanteur 30 avec de l'eau 32. La phase organique 33 est recyclée au plateau 22 de la colonne 3 et la phase aqueuse 34 est envoyée à un traitement biologique. La décantation dans le décanteur 30 est réalisée avec 20% d'eau par rapport au flux 31 à une température de 70°C.

On constate que le traitement du flux 31 soutiré latéralement permet de réduire la teneur en acides (AHP + Di-AA) à 58 ppm tout en respectant la spécification globale en A2EH à 99,7% pour le flux 7 extrait en tête de colonne 6 (voir tableau 4).

Par ailleurs le coût énergétique de cette opération n'est que de 1% supérieur au coût de la mise en oeuvre de l'exemple 1 comparatif, pour une production accrue à 124T/j.

**[Tableau 4]**

| Flux | 2 | 4 | 5 | 7 | 8 | 11 | 31 | 33 | 34 |
|---|---|---|---|---|---|---|---|---|---|
| | | | | | | | | | |
| Température °C | 115 | 29,7 | 143,1 | 23,8 | 144,2 | 138 | 138,9 | 70 | 70 |
| Débit massique, kg/hr | 13600 | 7365,358 | 6500 | 5149,999 | 1350 | 319,548 | 1000 | 995,525 | 204,475 |
| Fraction massique | | | | | | | | | |
| AA | 0,065 | 0,122 | 0 | 0 | 0 | 0 | 0 | 0 | 0 |
| 2EH | 0,23 | 0,434 | 0 | 0 | 0 | 0 | 0,002 | 0,002 | 0 |
| A2EH | 0,649 | 0,389 | 0,943 | 0,997 | 0,74 | 0,326 | 0,95 | 0,954 | 0,001 |
| EAU | 0,005 | 0,009 | 0 | 0 | 0 | 0 | 0 | 0,005 | 0,953 |
| Di-AA | 0,001 | 0 | 0,001 | 29 | 0,007 | 0,006 | 0 | 0 | 0,001 |
| AHP, ppm | 140 | 44 | 25 | 29 | 9 | 8 | 2000 | 62 | 0,007 |

### Exemple 5 (selon l'invention)

L'exemple 4 est reproduit en changeant certaines conditions opératoires, avec une production en A2EH maintenue à 119T/j et la teneur en AHP dans le flux d'alimentation 2 fixée à 140 ppm.

### - Effet de la température de décantation du décanteur 30

La température de décantation dans un domaine de 20°C à 70°C n'a pas d'influence sur les performances de ce traitement (tableau 5).

**[Tableau 5]**

| | | | |
|---|---|---|---|
| T° de décantation (°C) | 20 | 50 | 70 |
| débit du flux 31 (kg/h) | 300 | 300 | 300 |
| débit du flux 32 (kg/h) | 60 | 60 | 60 |
| production du flux 7 (t/j) | 119 | 119 | 119 |
| Energie mise en oeuvre (kcal/h) | 2239082 | 2235646 | 2233537 |
| flux 7 (fraction massique) | | | |
| AA, ppm | 0 | 0 | 0 |
| A2EH, % | 99,7 | 99,7 | 99,7 |
| di-AA, ppm | 28 | 28 | 28 |
| AHP, ppm | 56 | 57 | 57 |

### - Effet du débit de soutirage latéral du flux 31

L'augmentation du débit du soutirage latéral 31 permet d'améliorer la teneur en acidité dans le flux purifié 7, sans toutefois accroître le niveau de pureté maintenu à 99,7% (tableau 6).

**[Tableau 6]**

| | | | |
|---|---|---|---|
| T° de décantation (°C) | 20 | 20 | 20 |
| débit du flux 31 (kg/h) | 300 | 1000 | 10000 |
| Débit du flux 32 (kg/h) | 60 | 200 | 60 |
| production du flux 7 (t/j) | 119 | 119 | 124 |
| Energie mise en oeuvre (kcal/h) | 2239082 | 2258023 | 2263410 |
| flux 7 (fraction massique) | | | |
| A2EH | 99,7 | 99,7 | 99,7 |
| AA, ppm | 0 | 0 | 0 |
| di-AA, ppm | 28 | 29 | 29 |
| AHP, ppm | 56 | 25 | 26 |

### - Effet de la quantité d'eau de lavage 32 introduite dans le flux 31

Le débit d'eau de lavage par rapport au débit du flux soutiré latéralement a peu d'influence sur le traitement de lavage (tableau 7).

**[Tableau 7]**

| | | |
|---|---|---|
| T° de décantation (°C) | 70 | 70 |
| débit du flux 31 (kg/h) | 300 | 300 |
| Débit du flux 32 (kg/h) | 30 | 60 |
| production du flux 7 (t/j) | 119 | 119 |
| Energie mise en oeuvre (kcal/h) | 2235646 | 2233537 |
| flux 7 (fraction massique) | | |
| A2EH | 99,7 | 99,7 |
| AA, ppm | 0 | 0 |
| di-AA, ppm | 28 | 28 |
| AHP, ppm | 59 | 57 |

## Revendications

1. Procédé de récupération/purification d'un ester acrylique en C₄-C₁₀, à partir d'un mélange réactionnel brut obtenu par estérification directe de l'acide acrylique par l'alcool correspondant, **caractérisé en ce qu'**un flux riche en impuretés acides telles que l'acide β-hydroxypropionique et l'acide β-acryloxypropionique est soutiré par une sortie latérale lors de la distillation du mélange réactionnel brut.

2. Procédé selon la revendication 1 **caractérisé en ce que** le flux riche en impuretés acides est soutiré sous forme gazeuse ou sous forme liquide, de préférence sous forme liquide.

3. Procédé selon la revendication 1 ou 2 **caractérisé en ce que** le soutirage latéral est effectué à un niveau plus bas que le niveau d'alimentation de la colonne de distillation par le mélange réactionnel brut.

4. Procédé selon l'une quelconque des revendications 1 à 3 **caractérisé en ce que** le flux riche en impuretés acides, soutiré latéralement, est soumis à un traitement avec de l'eau, et le flux traité est recyclé dans la colonne de distillation.

5. Procédé selon la revendication 4 **caractérisé en ce que** le flux traité est recyclé dans la colonne de distillation à un niveau plus haut que le soutirage latéral.

6. Procédé selon la revendication 4 ou 5 **caractérisé en ce que** le flux traité est recyclé dans la colonne de distillation à un niveau plus bas que le niveau d'alimentation de la colonne de distillation.

7. Procédé de récupération/purification d'un ester acrylique en C₄-C₁₀, à partir d'un mélange réactionnel brut obtenu par estérification directe de l'acide acrylique par l'alcool correspondant, comprenant au moins les étapes suivantes :
i) on soumet le mélange réactionnel à un étêtage dans une colonne de distillation équipée d'un soutirage latéral permettant d'obtenir :
- en tête un flux composé essentiellement des réactifs non réagis ;
- en pied un flux comprenant l'ester recherché et des sous-produits lourds ;
- par soutirage latéral, un flux riche en impuretés acides ;
ii) on soumet le flux de pied de la colonne d'étêtage à une colonne de rectification permettant de séparer :
- en tête l'ester recherché purifié ;
- en pied un flux contenant des sous-produits lourds, qui est concentré sur un évaporateur à film ou distillé dans une colonne d'équeutage afin de recycler à la colonne d'étêtage les composés légers présents, et d'éliminer un résidu final de sous-produits lourds.

8. Procédé selon revendication 7 comprenant en outre une étape iii) de traitement du flux soutiré latéralement :
iii) on soumet le flux riche en impuretés acides à une étape de lavage avec un flux aqueux permettant d'obtenir après décantation,
- une phase aqueuse comprenant la totalité des impuretés acides qui peut être envoyée à une station de traitement biologique ou en partie utilisée comme flux aqueux de lavage, et
- une phase organique, comprenant l'ester recherché, des sous-produits lourds et des traces d'eau et de réactifs, qui est recyclée au moins en partie dans la colonne d'étêtage.

9. Procédé selon l'une quelconque des revendications précédentes **caractérisé en ce que** l'ester acrylique est l'acrylate de 2-éthylhexyle.

10. Procédé de production d'un ester acrylique en C₄-C₁₀ exempt d'impuretés acides par estérification directe de l'acide acrylique par l'alcool correspondant comprenant le procédé de récupération/purification tel que défini selon l'une quelconque des revendications précédentes.

## Patentansprüche

1. Verfahren zur Gewinnung/Reinigung eines C₄-C₁₀-Acrylsäureesters aus einer rohen Reaktionsmischung, die durch direkte Veresterung von Acrylsäure mit dem entsprechenden Alkohol erhalten wird, **dadurch gekennzeichnet, dass** bei der Destillation der rohen Reaktionsmischung ein Strom, der reich an sauren Verunreinigungen wie β-Hydroxypropionsäure und β-Acryloxypropionsäure ist, an einem seitlichen Auslass entnommen wird.

2. Verfahren nach Anspruch 1, **dadurch gekennzeichnet, dass** der an sauren Verunreinigungen reiche Strom in Gasform oder in flüssiger Form, vorzugsweise in flüssiger Form, entnommen wird.

3. Verfahren nach Anspruch 1 oder 2, **dadurch gekennzeichnet, dass** die seitliche Entnahme auf einer Höhe unterhalb der Höhe der Zuführung der rohen Reaktionsmischung zur Destillationskolonne erfolgt.

4. Verfahren nach einem der Ansprüche 1 bis 3, **dadurch gekennzeichnet, dass** der seitlich entnommene, an sauren Verunreinigungen reiche Strom einer Behandlung mit Wasser unterzogen wird und der behandelte Strom in die Destillationskolonne zurückgeführt wird.

5. Verfahren nach Anspruch 4, **dadurch gekennzeichnet, dass** der behandelte Strom auf einer Höhe in die Destillationskolonne zurückgeführt wird, die höher als die seitliche Entnahme ist.

6. Verfahren nach Anspruch 4 oder 5, **dadurch gekennzeichnet, dass** der behandelte Strom auf einer Höhe unterhalb der Höhe der Zuführung zur Destillationskolonne in die Destillationskolonne zurückgeführt wird.

7. Verfahren zur Gewinnung/Reinigung eines C₄-C₁₀-Acrylsäureesters aus einer rohen Reaktionsmischung, die durch direkte Veresterung von Acrylsäure mit dem entsprechenden Alkohol erhalten wird, das zumindest die folgenden Schritte umfasst:
i) ein Einwirkenlassen eines Abtoppens auf die Reaktionsmischung in einer mit einer seitlichen Entnahme ausgestatteten Destillationskolonne, wodurch Folgendes erhältlich ist:
- am Kopf ein Strom, der im Wesentlichen aus nicht umgesetzten Reagenzien besteht;
- am Fuß ein Strom, der den gewünschten Ester und schwere Nebenprodukte umfasst;
- durch seitliche Entnahme ein an sauren Verunreinigungen reicher Strom;
ii) ein Einwirkenlassen einer Rektifikationskolonne auf den Bodenstrom der Abtoppkolonne, wodurch Folgendes trennbar ist:
- am Kopf der gewünschte gereinigte Ester;
- am Fuß ein schwere Nebenprodukte enthaltender Strom, der auf einem Dünnschichtverdampfer konzentriert oder in einer Nachlaufkolonne destilliert wird, um die vorhandenen leichten Verbindungen zur Abtoppkolonne zurückzuführen und einen endgültigen Rückstand von schweren Nebenprodukten zu entfernen.

8. Verfahren nach Anspruch 7, das weiterhin einen Schritt iii) des Behandelns des seitlich entnommenen Flusses umfasst:
iii) ein Einwirkenlassen eines Waschschritts mit einem wässrigen Strom auf den an sauren Verunreinigungen reichen Strom, wodurch nach einer Dekantierung Folgendes erhältlich ist,
- eine die Gesamtheit der sauren Verunreinigungen umfassende wässrige Phase, die einer biologischen Kläranlage zugeführt oder teilweise als wässriger Waschstrom verwendet werden kann, und
- eine organische Phase, die den gewünschten Ester, schwere Nebenprodukte und Spuren von Wasser und Reagenzien umfasst, die zumindest teilweise in die Abtoppkolonne zurückgeführt wird.

9. Verfahren nach einem der vorhergehenden Ansprüche, **dadurch gekennzeichnet, dass** es sich beim Acrylsäureester um 2-Ethylhexylacrylat handelt.

10. Verfahren zur Herstellung eines C₄-C₁₀-Acrylsäureesters, der frei von sauren Verunreinigungen ist, durch direkte Veresterung von Acrylsäure mit einem entsprechenden Alkohol, welches das Verfahren zur Gewinnung/Reinigung gemäß der Definition in einem der vorhergehenden Ansprüche umfasst.

## Claims

1. A process for the recovery/purification of a C₄-C₁₀ acrylic ester from a crude reaction mixture obtained by direct esterification of acrylic acid by the corresponding alcohol, **characterized in that** a stream rich in acid impurities, such as β-hydroxypropionic acid and β-acryloyloxypropionic acid, is drawn off via a side outlet during the distillation of the crude reaction mixture.

2. The process as claimed in claim 1, **characterized in that** the stream rich in acid impurities is drawn off in gaseous form or in liquid form, preferably in liquid form.

3. The process as claimed in claim 1 or 2, **characterized in that** the side draw-off is carried out at a lower level than the level of feeding of the distillation column by the crude reaction mixture.

4. The process as claimed in any one of claims 1 to 3, **characterized in that** the stream rich in acid impurities, drawn off laterally, is subjected to a treatment with water, and the treated stream is recycled in the distillation column.

5. The process as claimed in claim 4, **characterized in that** the treated stream is recycled in the distillation column at a higher level than the side draw-off.

6. The process as claimed in claim 4 or 5, **characterized in that** the treated stream is recycled in the distillation column at a lower level than the feed level of the distillation column.

7. A process for the recovery/purification of a C₄-C₁₀ acrylic ester, from a crude reaction mixture obtained by direct esterification of acrylic acid by the corresponding alcohol, comprising at least the following stages:
i) the reaction mixture is subjected to topping in a distillation column equipped with a side draw-off making it possible to obtain:
- at the top, a stream composed essentially of unreacted reactants;
- at the bottom, a stream comprising the desired ester and heavy byproducts;
- by side draw-off, a stream rich in acid impurities;
ii) the bottom stream from the topping column is subjected to a rectification column making it possible to separate:
- at the top, the purified desired ester;
- at the bottom, a stream containing heavy byproducts, which is concentrated on a film evaporator or distilled in a tailing column in order to recycle, to the topping column, the light compounds present and to remove a final residue of heavy byproducts.

8. The process as claimed in claim 7, additionally comprising a stage iii) of treatment of the stream drawn off laterally:
iii) the stream rich in acid impurities is subjected to a stage of washing with an aqueous stream making it possible to obtain, after separation by settling,
- an aqueous phase comprising all of the acid impurities, which can be sent to a biological treatment plant or in part used as aqueous washing stream, and
- an organic phase comprising the desired ester, heavy byproducts and traces of water and of reagents, which is recycled at least in part in the topping column.

9. The process as claimed in any one of the preceding claims, **characterized in that** the acrylic ester is 2-ethylhexyl acrylate.

10. A process for the production of a C₄-C₁₀ acrylic ester devoid of acid impurities by direct esterification of acrylic acid by the corresponding alcohol comprising the recovery/purification process as defined according to any one of the preceding claims.
